# EUROPEAN PATENT APPLICATION

(11) **EP 4 556 547 A1**
(43) Date of publication of application: **21.05.2025**
(21) Application number: 23209847.5
(22) Date of filing: 14.11.2023
(51) Int. Cl.: C10G 2/00, C07C 29/15, C10G 31/08, C10K 3/02, C25B 1/04, B01D 53/14

(54) **PROCESS OF PRODUCING A FUEL**

(71) Applicant: SCW Systems B.V., 1812 PS Alkmaar (NL)
(72) Inventor: Essing, Gerard, 1812 PS Alkmaar (NL); Hauwert, Peter, 1812 PS Alkmaar (NL); Ariens, Maxim, 1812 PS Alkmaar (NL); Rommens, Hidde, 1812 PS Alkmaar (NL); Korndorfer, Frans, 1812 PS Alkmaar (NL)
(74) Representative: De Vries & Metman

(57) **Abstract**

Process for the production of a fuel. In a conversion step carbon dioxide is reacted with hydrogen to form a liquid carrier. The carbon dioxide is for instance collected with a direct air capture system. The hydrogen can for example be generated using renewable sources. After storage and transport to a site of use, the liquid carrier is mixed with water to form a ready mix. During a break-up step, the liquid carrier is converted to a fuel while the temperature and the pressure of the ready mix are maintained at sub- or supercritical conditions.

## Description

The invention relates to a process for the production of a fuel, in particular green hydrogen, green carbon monoxide and/or green methane. In a conversion step carbon dioxide is reacted with hydrogen to form a liquid carrier. In a break-up step, after storage and transport of the liquid carrier, the liquid carrier is converted to a desired fuel or mixture of fuels.

Most hydrogen is so-called grey hydrogen produced from fossil fuels, causing carbon dioxide emissions. To avoid the use of fossil fuels, hydrogen can be made by electrolysis using electricity from renewable sources, in particular wind energy or solar energy. Hydrogen produced with this technology is commonly referred to as green hydrogen.

Green hydrogen is typically produced by electrolysis at locations where electricity from renewable sources is abundantly available, especially at locations with very sunny or windy weather conditions, close to wind farms or solar parks. From there, the hydrogen is transported to locations of use.

Due to its very low density hydrogen can be liquefied for storage and transport. Liquefied hydrogen improves on storage density but is challenging to store and transport without significant boil-off losses. Alternatively, to save energy needed for liquefaction, hydrogen carriers can be used for storage of hydrogen as a liquid, such as liquid organic hydrogen carriers (LOHCs). LOHCs are organic unsaturated compounds that can absorb hydrogen by hydrogenation and release hydrogen by dehydrogenation. The sequence of endothermal dehydrogenation followed by hydrogen purification limits the overall efficiency. The LOHC's must be transported back and forth between the location of hydrogenation and the location of dehydrogenation, which also impacts the efficiency of this storage cycle.

Ammonia is a possible hydrogen carrier which is relatively stable and easy to transport. However, the energy costs of dehydrogenation are high. Using ammonia as a hydrogen carrier offers low return on energy investment and also poses health risks.

Also formic acid can be used as a hydrogen carrier, particularly due to its high volumetric capacity (53 g H2/L) and its low toxicity and flammability under ambient conditions. The article *"Formic Acid as a Hydrogen Energy Carrier"* discloses a method wherein ambient carbon dioxide is captured and hydrogenated to form formic acid. The hydrogen is produced by electrolysis using energy from renewable sources. The formic acid can be transported via existing infrastructure to locations where it can be dehydrogenated to produce hydrogen and carbon dioxide. Subsequently, the hydrogen needs to be pressurized before it can be used as a fuel. Other fuels, such as methane, cannot be produced with this system. Another drawback is that the energy density of formic acid is low (5.5 MJ/kg).

It is an object of the present invention to provide a more flexible and economic process for safe transport, storage and/or use of hydrogen and/or other fuels derived from the process.

The object of the invention is achieved with a process for the production of a fuel. In a conversion step carbon dioxide is reacted with hydrogen to form a liquid carrier. After storage and transport, the liquid carrier is converted into a fuel in a break-up step. Before the break-up step the liquid carrier is mixed with water to form an aqueous ready mix. During the break-up step the temperature and the pressure of the ready mix are maintained at least at subcritical conditions, e.g., at supercritical conditions, i.e., such conditions that at least the water content of the ready mix is in a supercritical state.

This process yields a pressurized fuel, avoiding the need for an additional pressurization step. The process is very flexible and can be used to produce a variety of fuels, including carbon monoxide, hydrogen and methane. The sub- or supercritical water gasification process can break down organic compounds into carbon monoxide, hydrogen, methane or other hydrocarbon fuel gases, so it provides substantial flexibility during the conversion step. Hence, the conversion step does not need to produce high purity yields of specific organic liquids, as would be the case if the produced liquid were used directly as a combustible fuel or as a chemical component for an industrial process. In the process of the present invention, carbon dioxide is not used as a merely passive carrier for hydrogen, but contributes to the calorific value of the end product through the formation of non-fossil methane and carbon monoxide. The energy value of the end product is therefore significantly higher than when using formic acid, ammonium or LOHCs as a hydrogen carrier. Although using the methane and carbon monoxide as fuel will regenerate carbon dioxide, the net emissions are zero because the production of these fuels uses the same amount of carbon dioxide. The carbon dioxide emissions can even be negative if the carbon dioxide is subsequently sequestered.

Water at subcritical conditions has a temperature above 100°C, but is pressurized to remain liquid. Under these conditions, water has a substantially lower polarity and less hydrogen bonds, resulting in an improved ability to dissolve non-polar compounds. These effects are further improved if the water is at supercritical conditions. Supercritical water is at a temperature and pressure above its thermodynamic critical point. More particularly, supercritical water has a temperature of at least 373°C and is at a pressure of at least 220 bar.

Hydrogen used in the conversion step can for example wholly or partly be produced by electrolysis using electricity from renewable sources, such as solar energy and/or wind energy. The electrolysis can be carried out close to the site where the renewable energy is produced. Hydrogen production can for example be used to store excess energy during power production peaks and to balance demand and supply on the power grid.

Water is fed to an electrolyzer to produce hydrogen by electrolysis. Electrolyzers comprise an anode and a cathode separated by an electrolyte. The anode or cathode can comprise catalysts. At the cathode water combines with electrons to form hydrogen and oxygen ions. The oxygen ions migrate to the anode where they react to form oxygen and release electrons. In this way, both hydrogen gas and oxygen gas are produced in the electrolyzer.

The electrolyzer can for example be an alkaline electrolyzer, membrane electrolyzer, and/or high temperature electrolyzer. Electrolysis can be used for multi-MW operation. Some electrolyzers are designed to operate at elevated pressures, e.g., 30-50 bar. This helps to avoid or reduce compression steps at a later stage of the process. Optionally, multiple electrolyzers can be operated in parallel.

Suitable electrolytes include for example aqueous solutions of KOH and/or NaOH. Activating compounds can be added to the electrolyte to improve the stability of the electrolyte.

Optionally, also the conversion step for hydrogenating carbon dioxide can be carried out close to the site where the renewable energy is produced.

In a specific embodiment, the carbon dioxide can wholly or partly be collected by direct air capture. The carbon dioxide is thus extracted from the atmosphere.

Several different Direct Air Capture (DAC) technologies can be used in the invention. The direct air capture technology can for example be based on a solid amine-based adsorbent, which is capable of chemisorbing carbon dioxide in the air that passes through the DAC reactor at near ambient temperatures and pressures.

The DAC reactor can for example be operated to remove about 20-50% of the carbon dioxide in the air passing through the DAC reactor. After the CO2 uptake cycle is complete, the DAC reactor is switched to the CO2 release adsorbent regeneration cycle.

Though carbon dioxide is released again by the break-up step at the end of the process, the carbon dioxide released at that stage can be sequestered, e.g., using magnesium silicates or similar magnesium and/or calcium containing compounds and the resulting carbonates can be used as a raw material, e.g., in cement industry. This way, the process can be carried out with negative carbon dioxide emissions.

The carbon dioxide can for example be hydrogenated using the Reverse Water Gas Shift (RWGS) reaction:

CO2 + H2 => CO + H2O

Accordingly, this reaction produces a mixture of carbon monoxide and water, generally called synthesis gas or syngas. The RWGS reaction is endothermic and requires a catalyst. Examples of suitable catalysts include Cu or Pt or Rh dispersed on metal oxide supports.

To start up the RWGS process, hydrogen produced by the electrolyser is mixed with carbon dioxide collected by the direct air capture, e.g., in a mixing ratio of H2 : CO2 between 2.0-5.0 mol/mol, e.g., between 3.0-4.0 mol/mol, to form an RWGS feed gas. The RWGS feed gas is heated, e.g., by a heat exchanger to a temperature above 750°C. Heating can for example be done by means of a heat exchanger and/or by electric heating using electricity from the aforementioned nearby renewable sources.

The heated RWGS feed gas then is fed into a RWGS reactor. A bed of catalyst is inside the RWGS reactor. Several catalyst materials are possible that can catalyse the RWGS reaction. Examples of suitable RWGS catalysts include Cu or Pt or Rh dispersed on metal oxide supports.

RWGS product gas is discharged from the RWGS reactor and comprises carbon monoxide (CO) and water (H2O), but also non-converted hydrogen (H2) and carbon dioxide (CO2), and possibly a small amount of methane (CH4). The water content of the RWGS product gas can for example be recycled to the electrolyser for the production of hydrogen. The other contents of the RWGS can be used for the production of the liquid carrier, for example by means of a Fischer-Tropsch process.

A Fischer-Tropsch process involves a number of chemical reactions converting carbon monoxide and hydrogen into a variety of hydrocarbons in gas, liquid or solid form, e.g. :

(2n + 1) H2 + n CO → CnH2n+2 + n H2O

where n is typically in the range of 1 to 60, e.g., 10 to 20. These reactions occur in the presence of metal catalysts, typically at temperatures of 150-300 °C and pressures of about 1 - 100 bar.

Catalysts for Fischer-Tropsch synthesis are known in the art and include for example cobalt based and/or iron based catalysts which can also be used for the RWGS reaction.

Optionally, carbon monoxide resulting from the RWGS process can be reacted with hydrogen from the electrolyser to form alcohols, in particular methanol, e.g.:

2 H2 + CO → CH3OH

In addition, ethanol, propanol, butanol and/or pentanol may be formed. Suitable catalysts are for example catalysts based on CuO. This reaction takes place at temperatures below 300°C and pressures below 50 bar.

In a specific embodiment, a mixture of hydrocarbons resulting from the Fischer-Tropsch process can be mixed with methanol produced by reacting hydrogen from the electrolyzer with carbon monoxide of the RWGS product gas.

It was found that a higher content of methanol promotes the yield of methane in the subsequent break-up process.

Optionally, the RWGS step can be omitted and carbon dioxide can be reacted directly with hydrogen to form alcohols. Suitable catalysts are for example catalysts based on Cu/ZnO/Al2O3. These reactions take place at temperatures below 300°C and pressures between 20 and 100 bar.

The liquid carriers produced with the processes set out above, are mixtures of hydrocarbons and/or alcohols with a relatively high energy density or calorific value (typically with an average energy density of about 20 to about 40 MJ/kg). The liquid carriers can safely be stored and transported using existing distribution infrastructure used for classic fossil fuels, such as existing tankers and pipelines. The process of the invention facilitates safe and efficient storage and transport, allowing utilization of the hydrogen at a location at long distance from the site where the hydrogen is produced. For example, the hydrogen can be produced in a place with sunny or windy weather conditions and then transported to a place where there is a need for hydrogen, methane and/or carbon monoxide as a fuel.

The break-up step can wholly or partly be carried out at long distance from the site where the conversion step was carried out. For example, before the break-up step the liquid carrier is mixed with water to form a ready mix. This mixing step can be carried out either at the site of the conversion step, or at the site of the break-up step where the sub- or supercritical water gasification takes place. The ready mix can have any suitable water content. The water content of the ready mix should be sufficient to allow gasification under supercritical conditions, but excess water can result in more energy consumption. An optimum water content can be determined in dependence of the residence time, process temperature and the nature and complexity of the constituents of the liquid carrier. The water content may for example be in the range of about 5 wt% to about 95 wt%, e.g., below 70 wt%, e.g., below 30 wt%, e.g., between 20 to 30 wt%.

The ready mix is subsequently pressurized and heated to bring its' water content in the desired sub- or supercritical state. Subcritical conditions already provide conversion to gaseous fuels, but results can improve with temperatures and a pressures closer to, or higher than, the thermodynamic critical point of water. The temperature of the aqueous mixture can be above 100°C, preferably above 300°C, more preferably above 350°C, while being at a pressure sufficient to maintain the water of the aqueous mixture in a liquid state. Supercritical conditions are preferred. This occurs when the temperature is at least 373°C and the temperature is at least 220 bar.

During the break-up step, a variety of processes take place. Process parameters, such as residence time, temperature and type of catalysts can be used to promote certain processes, in order to promote production of a specific fuel. For example, using a suitable RWGS catalyst, promotes RWGS processes and the formation of carbon monoxide. Also methanation catalysts, e.g., nickel alloys catalyse hydrogenation reactions and promote methanation.

The break-up process is an anaerobe and endothermic gasification process, generally referred to as Supercritical Water Gasification (SCWG), to be distinguished from Supercritical Water Oxidation (SCWO) which is an exothermic process requiring the addition of effective amounts of an oxidizing agent. Hitherto, SCWG processes are used for the breakdown of biomass or plastic or similar organic compounds to produce combustible fuel gases, such as hydrogen, methane and/or carbon monoxide.

Although catalysts can be used to promote the formation of specific constituents, the break-up process will typically comprise a mixture of hydrogen, carbon dioxide, carbon monoxide, and methane. The mixture can be further processed to provide fuels of the desired purity. For example, the mixture can be subjected to a methanation process converting hydrogen, carbon dioxide, and carbon monoxide to methane. A steam reforming process can be used to convert methane to syngas comprising hydrogen and carbon monoxide, which can be separated to provide hydrogen of a desired purity.

At the end of the break-up process, carbon dioxide can also be formed if one or more components of the liquid carrier comprises oxygen atoms. This carbon dioxide is separated from the yielded fuel gases. In a subsequent sequestration step, the carbon dioxide, preferably still in a pressurized state, can be reacted with one or more sequestering compounds, e.g., a silicate containing a divalent metal, such as magnesium, in particular a nesosilicate. Particularly suitable sequestering compounds include for example serpentine, olivine, brucite, pyroxene, magnesium oxide, magnesium hydroxide and calcium hydroxide, or mixtures thereof. Exemplary processes for the sequestration of carbon dioxide are disclosed in US 2005/0180910, US 2022/0250917 and EP 2 511 003. This sequestration prevents emission of carbon dioxide.

The sequestering step can be used to produce compounds, in particular carbonates and/or silicates, which can be used as raw materials, e.g., in the production of cement. This contributes to the reduction of emission of carbon dioxide by the cement industry.

In this way, the process as a whole not only captures and permanently retains carbon dioxide, but also prevents carbon dioxide emissions from the use of fossil fuels and also reduces carbon dioxide emissions from the cement industry.

### Example

To produce an amount of a gaseous fuel with a total energetic value of 100 MW, carbon dioxide is captured using a direct air capture system and mixed with 30,000 - 40,000 ton hydrogen in a stoichiometric mixing ratio to form an RWGS feed gas. The RWGS feed gas is then heated to a temperature above 750°C and fed to an RWGS reactor with a catalyst bed of an RWGS catalyst. This produces a RWSG product gas mainly comprising H2O and CO. The H2O is separated and recycled to feed the electrolyser. The CO is fed to a Fischer Tropsch reactor at a temperature of 300°C and a pressure of 100 bar with an effective amount of a catalyst. This results in a liquid carrier comprising a mixture of hydrocarbons including octane, nonane, decane, undecane dodecane, tridecane, tetradecane pentadecane and hexadecane. This mixture of hydrocarbons has a calorific value of more than 35 MJ/kg.

In a next step, the liquid carrier is mixed with water to prepare a ready mix with a water content of about 20 - 50 wt%. This ready mix is then pressurized to at least 220 bar and heated to a temperature of at least 373°C, i.e., at supercritical conditions. After a residence time of at least 10 seconds the gaseous constituents are separated. This mixture has a total energetic value of about 100 MW.

### Comparative Example

To produce an amount of fuel with a total energetic value of 100 MW, while using formic acid as a liquid carrier, a total of more than 250,000 tons of hydrogen are produced in an electrolyser fed by renewable sources. Carbon dioxide is captured using a direct air capture system and mixed with hydrogen in a stoichiometric mixing ratio. This mixture is then fed to a reactor comprising a catalyst for the endothermic synthesis of formic acid. This results in 730,000 tons of formic acid, which has a calorific value of approximately 5,5 MJ/kg. At a later stage the formic acid is dehydrogenated.

The examples show that with the process of the present invention substantially less hydrogen is needed as well as significantly less liquid carrier to produce an amount of renewable combustible gas product with the same energetic value. With prior art systems, the liquid carrier is merely dehydrogenated and does not add to the energetic value of the final product gas. With the present invention, the hydrogenated CO2 is also at least partly converted to combustible fuels (methane, carbon monoxide) so it does contribute to the energetic value of the final product gas.

In the context of the present disclosure, the word fuel refers to combustible compounds that can be used as fuel, but other uses of these substances are not excluded from the scope of the present invention. Hydrogen and/or methane extracted using the process according to the invention can also be used, for example, as raw material for other chemical processes within the scope of the invention.

## Claims

1. Process for the production of a fuel, wherein:
in a conversion step carbon dioxide is reacted with hydrogen to form a liquid carrier;
in a break-up step after storage and transport of the liquid carrier, the liquid carrier is converted to a fuel;
**characterized in that** before the break-up step the liquid carrier is mixed with water to form a ready mix,
wherein during the break-up step the temperature and the pressure of the ready mix are maintained at sub- or supercritical conditions.

2. Process according to claim 1, wherein the hydrogen is at least partly produced by electrolysis of water using electricity from renewable sources.

3. Process according to claim 1 or 2, wherein the carbon dioxide is at least partly collected by direct air capture.

4. Process according to any one of the preceding claims, wherein the conversion step includes a hydrogenation step, in which the carbon dioxide is hydrogenated to produce carbon monoxide and water, and a subsequent synthesis step in which the carbon monoxide is hydrogenated to form the liquid carrier.

5. Process according to claim 2 and 4, wherein the water generated in the hydrogenation step is recycled to an electrolyser to produce hydrogen.

6. Process according to claim 4 or 5, wherein the synthesis step includes a Fischer-Tropsch process.

7. Process according to claim 4 or 5, wherein the synthesis step includes hydrogenation of at least a part of the carbon monoxide to form alcohols, in particular methanol.

8. Process according to any one of the preceding claims, wherein the conversion step includes a direct hydrogenation step, wherein carbon dioxide is directly hydrogenated to produce one or more alcohols, in particular methanol.

9. Process according to claim 6, 7 or 8, wherein alcohol, e.g., formed by the step of claim 7 or 8, is mixed with the liquid carrier formed with the Fischer-Tropsch process.

10. Process according to any one of the preceding claims, wherein the break-up step involves a sub- or supercritical water gasification process.

11. Process according to anyone of the preceding claims, wherein hydrogen and/or methane produced in the break-up step are separated and prepared for use as a fuel, e.g., by a methanation or steam reform process.

12. Process according to anyone of the preceding claims, wherein carbon dioxide is separated in the break-up step and subsequently reacted with a sequestering compound.

13. Process according to claim 12, wherein the carbon dioxide and the sequestering compound react to produce a component for the production of cement.
